# FASCICULE DE BREVET EUROPEEN

(11) **EP 0 456 534 B1**
(45) Date de publication et mention de la délivrance du brevet: **26.01.1994**
(21) Numéro de dépôt: 91400962.6
(22) Date de dépôt: 10.04.1991
(51) Int. Cl.: A61F 5/01, F16B 11/00

(54) **Système d'assemblage d'une orthèse et procédé de réalisation**
System und Verfahren zum Zusammenbau einer Orthese
Orthosis assembly system and method for its production

(30) Priorité: 10.05.1990 FR 9005817
(43) Date de publication de la demande: 13.11.1991
(73) Titulaire: ETABLISSEMENTS PROTEOR, 21100 Dijon (FR)
(72) Inventeur: Vera, Bernard, F-21250 Seurre (FR)
(74) Mandataire: Jolly, Jean-Pierre

(56) Documents cités:
- DE-A- 3 635 031
- US-A- 4 502 472
- US-A- 4 561 670

## Description

La présente invention concerne un nouveau système d'assemblage d'un montant et d'une articulation d'une orthèse des membres supérieurs ou inférieurs. L'invention concerne également un procédé pour la réalisation d'un tel système d'assemblage.

On sait que de telles orthèses, utilisées au niveau du coude ou du genou des patients, comportent généralement deux montants longitudinaux, disposés à l'extérieur de chacun des membres (bras et avant-bras, ou jambe et cuisse) et réunis par des entretoises incurvées ou "embrasses" épousant le profil transversal du membre, chacun des montants associés aux bras et avant-bras ou à la cuisse et à la jambe étant articulés entre eux par une articulation comportant deux branches pivotantes autour d'un axe, cette articulation comportant éventuellement un élément de verrouillage en position.

Les deux branches de l'articulation ont souvent la forme d'un fourreau, dans lequel est engagée l'extrémité correspondante du montant associé, la branche de l'articulation et le montant étant rendus rigidement solidaires l'un de l'autre à l'aide d'au moins une vis.

Avant d'être rendus solidaires de l'articulation, chaque montant doit être orienté avec une grande précision par rapport à l'axe de celle-ci et, lorsqu'on emboîte simplement l'extrémité du montant dans le fourreau associé de l'articulation, la mise en position résultante est très souvent d'une précision insuffisante.

La présente invention vise à remédier à cet inconvénient en proposant un système d'assemblage en une position relative précise des montants et des articulations d'une telle orthèse.

L'invention a également pour but de proposer un système de ce type qui puisse être réalisé facilement à l'aide de moyens usuels dans la technique.

L'invention vise enfin à proposer des orthèses comportant un tel système d'assemblage et présentant une grande résistance mécanique au niveau de l'assemblage des montants et des articulations.

A cet effet, l'invention a pour objet un système d'assemblage d'un montant d'une orthèse des membres inférieurs ou supérieurs et d'une branche d'une articulation à deux branches montées pivotantes autour d'un axe l'une par rapport à l'autre, système dans lequel la branche de l'articulation forme un fourreau dans la cavité duquel est engagée l'extrémité du montant associé, cette extrémité et la branche du fourreau étant rendues rigidement solidaires l'une de l'autre par au moins une vis disposée perpendiculairement aux faces latérales des montants, ce système d'assemblage étant caractérisé en ce que l'extrémité du montant engagé dans la cavité du fourreau n'est pas en contact avec le fond de cette cavité, en ce que ses grandes faces ont une largeur inférieure à celle de la dimension correspondante de la cavité du fourreau, tandis que les mêmes faces de ce montant extérieures à cette cavité ont une largeur supérieure à celle de la dimension correspondante de l'ouverture du fourreau, afin que le montant prenne appui latéralement par ses flancs perpendiculaires aux grandes faces contre les parties correspondantes de l'ouverture du fourreau, et en ce que l'extrémité du montant engagée dans la cavité du fourreau est noyée dans un matériau adhésif solide emplissant cette cavité et adhérant aux surfaces internes de celle-ci et aux faces externes de l'extrémité du montant.

Le montant de l'orthèse et la branche associée de l'articulation sont donc solidarisés, d'une part, par la vis de la technique antérieure, d'autre part, par le matériau adhésif solide, par exemple un polymère, une colle ou un ciment emplissant la cavité du fourreau. Du fait de cette double solidarisation, aucune contrainte de rupture ne s'exercera sur la vis. Par ailleurs, le montant venant buter latéralement par ses flancs contre les parties correspondantes de l'ouverture de la cavité alors que son extrémité ne touche pas le fond de cette cavité, il sera possible de l'orienter avec précision par rapport à la branche du fourreau, avant de le solidariser de celle-ci à l'aide d'au moins une vis et d'au moins un monomère liquide ou pâteux (une résine) dont on provoque ensuite la polymérisation.

C'est ce qui ressort du procédé suivant de réalisation d'un tel système d'assemblage, qui constitue un autre objet de l'invention et qui comprend les phases successives suivantes :
- on engage dans la cavité de la branche formant fourreau de l'articulation, éventuellement percée transversalement d'un trou de vis, une extrémité du montant dont la largeur des grandes faces est inférieure à la dimension correspondante de la cavité, alors que les mêmes faces du montant extérieures à la cavité ont une largeur supérieure à la dimension correspondante de l'ouverture du fourreau, afin que le montant prenne appui par ses flancs perpendiculaires aux grandes faces contre les parties correspondantes de l'ouverture de la cavité, les dimensions de la partie engagée dans le fourreau étant telles que l'extrémité du montant ne touche pas le fond de la cavité ;
- on oriente avec précision le montant par rapport à la branche de l'articulation ;
- on perce des trous de vis disposés en regard dans le montant maintenu dans cette position et dans la branche formant fourreau de l'articulation, ou dans le montant seulement en regard du trou de vis que comporte déjà éventuellement la branche formant fourreau ;
- on retire du fourreau l'extrémité du montant ;
- on remplit la cavité du fourreau d'au moins un produit adhésif liquide ou pâteux, apte à durcir en formant un produit solide adhérant aux faces internes de la dite cavité ;
- on introduit à nouveau dans cette cavité l'extrémité du montant en éliminant de la cavité le trop-plein de produit liquide ou pâteux ;
- on amène en regard les trous de vis dont sont percés le montant et la branche formant fourreau de l'articulation ;
- on assemble par une vis engagée dans ces trous de vis l'extrémité du montant et la branche formant fourreau;
- on provoque le durcissement du produit liquide ou pâteux.

Toutes ces opérations sont faciles à mettre en oeuvre et l'on peut donc ainsi réaliser une orthèse des membres inférieurs ou des membres supérieurs dont les montants sont orientés avec précision par rapport à l'articulation et qui présente une excellente résistance mécanique au niveau de l'assemblage des montants et de l'articulation.

Les dessins shématiques annexés illustrent une forme de mise en oeuvre de l'invention . Sur ces dessins :
La figure 1 est une vue partielle en élévation d'une extrémité d'un montant d'une orthèse engagée dans la cavité d'une branche formant fourreau d'une articulation de cette orthèse, en vue d'orienter avec précision ce montant par rapport à l'articulation ;
La figure 2 est une coupe selon la ligne II - II de la figure 1 ;
Les figures 3 et 4 sont des vues analogues après assemblage, conformément à l'invention, de l'extrémité du montant et de la branche formant fourreau de l'articulation.

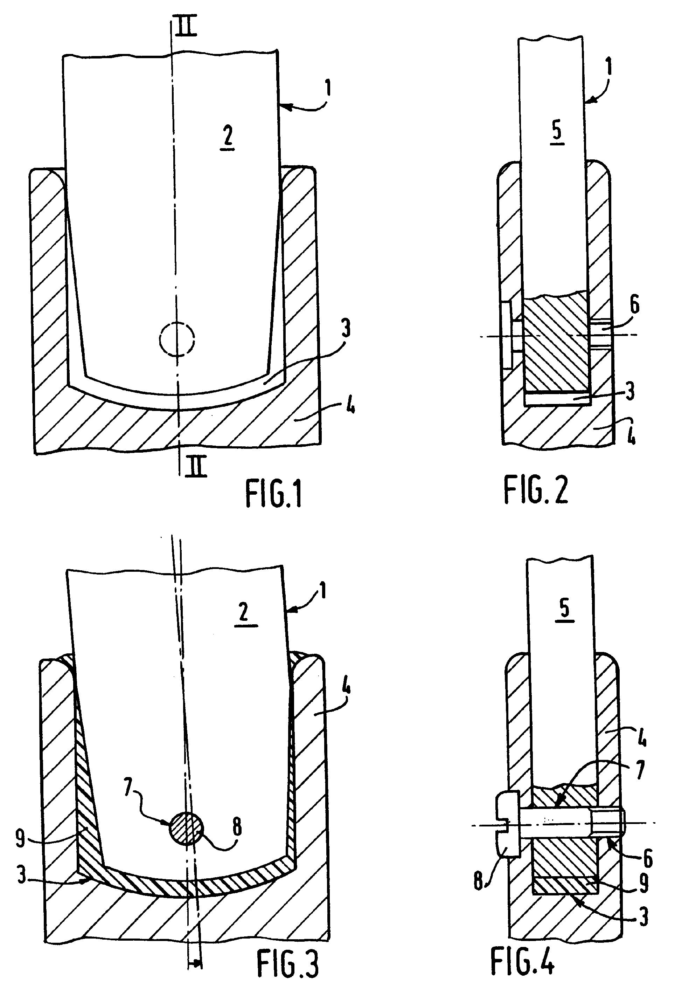

Comme on le voit sur les figures 1 et 2, l'extrémité d'un montant d'une orthèse comportant deux grandes faces parallèles 2 est engagée dans la cavité 3 d'une branche 4 formant fourreau d'une articulation de cette orthèse comportant deux branches analogues, articulées entre elles par un axe de rotation (non représenté) perpendiculaire aux grandes faces 2 des montants.

L'extrémité du montant 1 a une largeur inférieure à celle de la cavité 3, tandis que la partie du montant extérieure à la cavité 3 a une largeur supérieure à celle de l'ouverture de cette cavité. Ce montant 1 peut ainsi prendre appui par ses flancs 5 contre les bords correspondants de l'ouverture de la cavité 3.

La dimension longitudinale de l'extrémité du montant engagée dans la cavité 3 est telle que cette extrémité ne touche pas le fond de la cavité. Il est ainsi possible de faire pivoter le montant 1 par rapport à la branche 4 pour l'orienter avec précision dans la position désirée, par exemple celle qui apparaît sur la figure 3.

La branche 4 est percée transversalement d'un trou de vis 6 et, lorsque le montant est dans la position souhaitée par rapport à la branche 4, on perce ce montant d'un trou de vis identique en regard du trou 6. Si la branche n'était pas percée à l'avance d'un trou de vis, on percerait simultanément le montant 1 et la branche 4 de trous de vis disposés en regard, après avoir amené le montant 1 dans la position désirée.

On retire ensuite l'extrémité du montant 1 de la cavité 3 et l'on remplit cette cavité d'un monomère apte à polymériser en donnant un polymère solide, qui adhère au matériau dont sont constitués le montant 1 et la branche 4. On peut utiliser, par exemple, une résine époxy chargée de particules d'alumine, de particules métalliques ou autres.

On réintroduit alors le montant 1 dans la cavité 3, en faisant déborder le trop-plein du monomère, que l'on élimine.

On amène les trous de vis 6 et 7 en regard l'un de l'autre, ce qui a pour effet de ramener le montant 1 dans la position précise souhaitée par rapport à la branche 4 de l'articulation, et, dans cette position (figure 3) , on solidarise le montant 1 et la branche 4 à l'aide d'une vis 8 vissée dans les trous 6 et 7.

On provoque enfin la polymérisation du monomère, et l'on obtient ainsi un double assemblage du montant 1 et de la branche de l'articulation, par la vis 8, d'une part , et par la polymére 9, d'autre part, ce qui assure une parfaite résistance mécanique de cet assemblage . On notera que, du fait du polymère, aucun effort de cisaillement ne s'exercera sur la vis 8.

Les moyens utilisés pour réaliser cet assemblage en une position relative définie avec précision du montant 1 de la branche 4 de l'articulation sont des moyens simples, bien connus dans la technique, et il n'en résulte aucune majoration du coût de l'orthèse ainsi réalisée.

## Revendications

1. Système d'assemblage d'un montant (1) d'une orthèse des membres inférieurs ou supérieurs et d'une branche (4) d'une articulation à deux branches montées pivotantes autour d'un axe l'une par rapport à l'autre, système dans lequel la branche (1) de l'articulation forme un fourreau, dans la cavité (3) duquel est engagée l'extrémité du montant associé (1), cette extrémité et la branche du fourreau étant rendues rigidement solidaires l'une de l'autre par au moins une vis (8) disposée perpendiculairement aux faces latérales des montants, ce système d'assemblage étant caractérisé en ce que l'extrémité du montant (1) engagée dans la cavité (3) du fourreau n'est pas en contact avec le fond de cette cavité, en ce que ses grandes faces (2) ont une largeur inférieure à celle de la dimension correspondante de la cavité du fourreau, tandis que les mêmes faces (2) de ce montant extérieures à cette cavité ont une largeur supérieure à celle de la dimension correspondante de l'ouverture du fourreau, afin que le montant (1) prenne appui latéralement par ses flancs (5) perpendiculaires aux grandes faces (2) contre les parties correspondantes de l'ouverture du fourreau, et en ce que l'extrémité du montant engagé dans la cavité (3) du fourreau est noyée dans un matériau adhésif solide (9) emplissant cette cavité et adhérant aux surfaces internes de celle-ci et aux faces externes de l'extrémité du montant(1).

2. Procédé pour la réalisation d'un système d'assemblage selon la revendication 1 d'un montant (1) d'une orthèse des membres inférieurs ou supérieurs et d'une branche (4) d'une articulation à deux branches montées pivotantes autour d'un axe l'une par rapport à l'autre, caractérisé par les phases successives suivantes:
- on engage dans la cavité (3) de la branche (4) formant fourreau de l'articulation, éventuellement percée transversalement d'un trou de vis (6), une extrémité du montant (1) dont la largeur des grandes faces (2) est inférieure à la dimension correspondante de la cavité (3), alors que les mêmes faces (2) du montant (1) extérieures à la cavité (3) ont une largeur supérieure à la dimension correspondante de l'ouverture du fourreau, afin que le montant prenne appui par ses flancs (5) perpendiculaires aux grandes faces (2) contre les parties correspondantes de l'ouverture de la cavité, les dimensions de la partie engagée dans le fourreau étant telles que l'extrémité du montant (1) ne touche pas le fond de la cavité (3) ;
- on oriente avec précision le montant (1) par rapport à la branche (4) de l'articulation ;
- on perce des trous de vis (7) disposés en regard dans le montant maintenu dans cette position et dans la branche formant fourreau de l'articulation, ou dans le montant seulement en regard du trou de vis (6) que comporte déjà éventuellement la branche formant fourreau ;
- on retire du fourreau l'extrémité du montant (1) ;
- on remplit la cavité (3) du fourreau d'au moins un produit liquide et pâteux apte à durcir en formant un produit solide adhérant aux faces internes de la dite cavité ;
- on introduit à nouveau dans cette cavité (3)l'extrémité du montant (1) en éliminant de la cavité le trop plein de produit liquide ou pâteux ;
- on amène en regard les trous de vis (6,7) dont sont percés le montant et la branche formant fourreau de l'articulation ;
- on assemble par une vis (8) engagée dans ces trous de vis l'extrémité du montant (1) et la branche (4) formant fourreau;
- on provoque le durcissement du produit liquide ou pâteux.

## Patentansprüche

1. System zur Verbindung einer Strebe (1) einer Prothese für die unteren oder oberen Gliedmaßen und eines Schenkels (4) eines Gelenkes mit zwei Schenkeln, die zueinander um eine Achse drehbar angeordnet sind, ein System, in dem der Schenkel (1) des Gelenkes eine Hülse bildet, in deren Hohlraum (3) das Ende der zugehörigen Strebe (1) eingeführt ist und dieses Ende und der Schenkel der Hülse mit Hilfe mindestens einer Schraube (8) fest miteinander verbunden werden, die senkrecht zu den Seitenflächen der Streben angeordnet ist, wobei dieses Verbindungssystem dadurch gekennzeichnet ist, daß das Ende der in den Hohlraum (3) der Hülse eingreifenden Strebe (1) den Boden dieses Hohlraumes nicht berührt, daß ihre großen Seiten (2) von einer geringeren Breite als das entsprechende Maß des Hohlraumes sind, während die gleichen Seiten (2) dieser Strebe außerhalb dieses Hohlraumes breiter als das entsprechende Maß der Öffnung der Hülse sind, damit die Strebe (1) mit ihren zu den großen Seiten (2) senkrechten Flanken (5) seitlich auf den entsprechenden Teilen der Öffnung der Hülse aufliegt, und dadurch, daß das Ende der in den Hohlraum (3) der Hülse eingreifenden Strebe in einen festen Klebstoff (9) eingelassen ist, der diesen Hohlraum ausfüllt und an dessen Innenflächen sowie den Außenflächen des Endes der Strebe (1) haftet.

2. Ein Verfahren zur Herstellung eines Verbindungssystems nach Anspruch 1 einer Strebe (1) einer Prothese der unteren oder oberen Gliedmaßen und eines Schenkels (4) eines Gelenkes mit zwei zueinander um eine Achse drehbar angeordneten Schenkeln, durch die folgenden, nacheinander auszuführenden Schritte gekennzeichnet:
- in den Hohlraum (3) des Schenkels (4), der die Hülse des Gelenkes bildet und durch die eventuell quer ein Loch für eine Schraube (6) verläuft, wird ein Ende der Strebe (1) eingeführt, dessen Breite der großen Seiten (2) kleiner als das entsprechende Maß des Hohlraumes (3) ist, während die gleichen Seiten (2) der Strebe (1) außerhalb des Hohlraumes (3) breiter als das entsprechende Maß der Öffnung der Hülse sind, damit die Strebe mit ihren Flanken (5) senkrecht zu den großen Seiten (2) auf den entsprechenden Teilen der Öffnung des Hohlraumes aufliegt, wobei die Abmessungen des in die Hülse eingeführten Teils so sind, daß das Ende der Strebe (1) den Boden des Hohlraumes (3) nicht berührt;
- die Strebe (1) wird in bezug auf den Schenkel (4) des Gelenkes präzise ausgerichtet;
- es werden gegenüberliegende Löcher für Schrauben (7) in die in dieser Position gehaltene Strebe und in den Schenkel, der die Hülse des Gelenkes bildet, oder nur in die Strebe gegenüber dem Loch für die Schraube (6) gebohrt, das eventuell bereits in dem Schenkel, der die Hülse bildet, enthalten ist;
- das Ende der Strebe (1) wird aus der Hülse gezogen;
- der Hohlraum (3) der Hülse wird mindestens mit einem flüssigen und breiartigen Produkt gefüllt, das zu einem festen Produkt aushärten kann, das an den Innenwänden des genannten Hohlraumes haftet;
- in diesen Hohlraum (3) wird das Ende der Strebe (1) erneut eingeführt, indem das überflüssige flüssige oder breiartige Produkt aus dem Hohlraum entfernt wird;
- die in die Strebe und den Schenkel, welche die Hülse des Gelenkes bilden, gebohrten Löcher (6,7) werden gegenüberliegend ausgerichtet;
- das Ende der Strebe (1) und des Schenkels (4), welche die Hülse bilden, werden mit einer in diese Löcher eingeführten Schraube (8) verbunden;
- das flüssige oder breiartige Produkt wird zum Aushärten gebracht.

## Claims

1. A system for assembling a vertical member (1) of an orthesis for the lower and upper limbs and one branch (4) of a joint with two branches mounted pivotally with respects to each other about an axis, in which system the branch (1) of the joint forms a sleeve in the cavity (3) of which there is engaged the end of the associated vertical member (1), this end and the sleeve branch being firmly and rigidly connected together by at least one screw (8) arranged perpendicularly to the lateral faces of the vertical members, this system of assembly being characterized in that the end of the vertical member (1) engaged in the cavity (3) of the sleeve is not in contact with the base of this cavity, in that its large faces (2) have a width smaller than that of the corresponding dimension of the sleeve cavity, while the same faces (2) of this vertical member external to this cavity have a width greater than that of the corresponding dimension of the sleeve opening, so that the vertical member (1) rests laterally via its flanks perpendicular to the large faces (2) against the corresponding parts of the sleeve opening, and in that the end of the vertical member engaged in the cavity (3) of the sleeve is buried in a solid adhesive material (9) filling this cavity and adhering to the internal surfaces thereof and the external faces of the end of the vertical member (1).

2. A process for manufacturing an assembly system according to claim 1 of a vertical member (1) of an orthesis for the lower or upper limbs and a branch (4) of a joint with two branches mounted pivotally with respect to each other about an axis, characterized by the following successive stages:
- one end of the vertical member (1) is engaged in the cavity (3) in the joint branch (4) forming a sleeve, which branch (4) is optionally pierced transversely with a screw hole (6), the width of the large faces (2) of said vertical member end being smaller than the corresponding dimension of the cavity (3), while the same faces (2) of the vertical member (1) external to the cavity (3) have a width greater than the corresponding dimension of the sleeve opening, so that the vertical member rests via its flanks (5) perpendicular to the large faces (2) against the corresponding parts of the opening of the cavity, the dimensions of the part engaged in the sleeve being such that the end of the vertical member (1) does not touch the base of the cavity (3);
- the vertical member (1) is oriented with precision with respect to the branch (4) of the joint;
- screw holes (7) are pierced opposite each other in the vertical member held in this position and in the branch of the joint forming the sleeve, or in the vertical member only opposite the screw hole (6) which the sleeve-forming branch optionally already contains;
- the end of the vertical member (1) is withdrawn from the sleeve;
- the cavity (3) of the sleeve is filled with at least one liquid, pasty product capable of hardening to form a solid product adhering to the internal faces of said cavity;
- the end of the vertical member (1) is reintroduced into this cavity (3), eliminating from the cavity the excess liquid or pasty product;
- the screw holes (6, 7) pierced in the vertical member and the sleeve-forming branch of the joint are positioned opposite each other;
- the end of the vertical member (1) and the sleeve-forming branch (4) are assembled by a screw (8) engaged in these screw holes;
- hardening of the liquid or pasty product is caused.
